# EUROPEAN PATENT APPLICATION

(11) **EP 1 743 592 A1**
(43) Date of publication of application: **17.01.2007**
(21) Application number: 06253685.9
(22) Date of filing: 13.07.2006
(51) Int. Cl.: A61B 19/00, G02B 27/01, G06F 1/16

(54) **A system for providing information to a surgeon**

(30) Priority: 15.07.2005 US 182350
(71) Applicant: DePuy Products, Inc., Warsaw, IN 46581 (US)
(72) Inventor: Revie, Ian, Boroughbridge, York YO51 9AX (GB); Kissling, Jürgen, 97076 Würzburg (DE); Morrow, David W., Fort Wayne, IN 46804 (US); Winter, Robin, 85609 Aschheim (DE); Warnock, Alex, 85622 Feldkirchen (DE); Guzman, Jose F., Fort Wayne, IN 46804 (US); Heldreth, Mark A., Mentone, IN 46539 (US)
(74) Representative: Belcher, Simon James

(57) **Abstract**

A system for providing information related to an orthopaedic surgical procedure to a surgeon (12) includes a user-worn computer (16) coupled with a heads-up display (14). The user-worn computer (16) and the heads-up display (14) cooperate to display orthopaedic surgical information to the surgeon (12). The user-worn computer (16) receives data from a first processing circuit and a second processing circuit. The first processing circuit (40) determines navigational data based on data signals received from navigation sensors (48). The second processing circuit (60) determines patient condition data based on data signals received from patient condition sensors (68). Additionally, the system may include one or more peripheral ports (28,90) for receiving a removable memory device (30).

## Description

The present disclosure relates generally to computer assisted surgery systems for use in the performance of orthopaedic procedures.

During the performance of typical orthopaedic surgical procedures, orthopaedic surgeons rely on a broad range of orthopaedic surgical information. Such orthopaedic surgical information may include pre-operative notes and diagrams, patient X-rays and historical data, navigational data, surgical procedure images, data obtained from various sensors, and other data related to the orthopaedic surgical procedure and/or patient. The orthopaedic surgical information is typically provided to the surgeon via a number of different information systems, which may not be communicatively linked to one another. Accordingly, the surgeon is often required to interact independently with each information system to obtain the desired information. For example, the surgeon may be required to view different monitors to view the individual data.

Additionally, orthopaedic surgeons often spend a considerable amount of time and effort preparing the pre-operative notes, diagrams, and surgical plans on a computer system remote from the healthcare facility where the orthopaedic surgical procedure is to be performed (e.g., a computer system located in the surgeon's office). Because these remote computer systems are typically not in communication with (i.e., not communicatively coupled to) the healthcare facility's data network, the pre-operative information is typically not directly accessible and must be uploaded or otherwise incorporated into the existing information systems located at the healthcare facility.

In one aspect, the invention provides a system for providing information related to an orthopaedic surgical procedure to a surgeon. The system may include a heads-up display and a user-worn computer. The heads-up display and the user-worn computer may be configured to be worn by the surgeon. The heads-up display and the user-worn computer may be communicatively coupled via a wired or wireless communication link and may cooperate to display information related to the orthopaedic surgical procedure to the surgeon. The system may also include a microphone coupled to the user-worn computer. The user-worn computer may be configured to display information related to the orthopaedic surgical procedure on the heads-up display in response to voice commands received from the surgeon via the microphone. The user-worn computer may also include a receiver, transmitter, or transceiver for receiving and transmitting data.

The system may also include a first processing circuit. The first processing circuit may include a receiver and may be configured to receive a first data signal from a navigation sensor. The navigation sensor may be any type of sensor configured to produce a data signal indicative of the location of the sensor and/or a structure, such as a bone of a patient or a surgical tool, coupled with the navigation sensor. The first processing circuit may also be configured to determine navigational data, such as relative location or direction of motion of the sensor, based on the data signal. The first processing circuit may also include a transmitter and be configured to transmit the navigational data to the user-worn computer. The first processing circuit may transmit the navigational data via wired or wireless communication.

The system may further include a second processing circuit. The second processing circuit may include a receiver and may be configured to receive a second data signal from a patient condition sensor. The patient condition sensor may be any type of sensor, other than a navigation sensor, configured to produce a data signal indicative of some type of information related to the orthopaedic surgical procedure or the patient The second processing circuit may also be configured to determine patient condition data, such as a pressure value, a bone fracture value, or physiological data related to the patient, based on the data signal. The second processing circuit may also include a transmitter and be configured to transmit the patient condition data to the user-worn computer. The second processing circuit may transmit the patient condition data via wired or wireless communication.

The first processing circuit and the second processing circuit may be embodied as separate systems such as separate computer systems. Alternatively, the first and second processing circuits may be embodied as a single computer system. Further, in some embodiments, the first and/or the second processing circuit may form a portion of the user-worn computer. Additionally, the user-worn computer, the first processing circuit, the second processing circuit, and/or the single computer system may include a peripheral port configured to receive a removable memory device such as a memory device including a flash memory device or a microdrive.

Accordingly, in another aspect, the invention provides a system for providing information related to an orthopaedic surgical procedure to a surgeon, the system comprising:
a heads-up display configured to be worn by the surgeon;
a user-worn computer configured to be worn by the surgeon and communicatively coupled to the heads-up display, the user-worn computer being configured to receive data via a receiver and display the data to the surgeon on the heads-up display;
a first processing circuit configured to receive a first data signal from a navigation sensor, determine navigational data based on the first data signal, and transmit the navigational data to the user-worn computer; and
a second processing circuit configured to receive a second data signal from a patient condition sensor, determine patient condition data based on the second data signal, and transmit the patient condition data related to the orthopaedic surgical procedure to the user-worn computer.

In a further aspect, the invention provides a system for providing information related to an orthopaedic surgical procedure to a surgeon, the system comprising:
a heads-up display configured to be worn by the surgeon;
a user-worn computer configured to be worn by the surgeon and communicatively coupled to the heads-up display, the user-worn computer configured to receive a first data signal from a navigation sensor and a second data signal from a patient condition sensor and transmit the first and second data signals; and
a processing circuit configured to receive the first and second data signals from the user-worn computer, determine navigational data based on the first data signal and patient condition data based on the second data signal, and transmit the navigational and patient condition data to the user-worn computer.

The invention also provides a system for providing information related to an orthopaedic surgical procedure to a surgeon, the system comprising:
a heads-up display configured to be worn by the surgeon;
a user-worn computer configured to be worn by the surgeon and wirelessly communicatively coupled to the heads-up display, the user-worn computer being configured to receive data via a receiver and display the data to the surgeon on the heads-up display;
at least one sensor configured to transmit a data signal; and
a processing circuit configured to receive the data signal, determine patient condition data based on the data signal, and transmit the patient condition data to the user-worn computer.

Preferably, the user-worn computer includes a peripheral port configured to receive a removable memory device.

Preferably, the navigation sensor is coupled to a bone of a patient and the navigational data is indicative of the location of the bone of the patient.

Preferably, the navigation sensor forms a portion of an orthopaedic surgical tool and the navigational data is indicative of a location of the orthopaedic surgical tool.

Preferably, the patient condition sensor includes a pressure sensor and the patient condition data includes data indicative of a pressure applied to the pressure sensor.

Preferably, the patient condition sensor includes a fracture monitoring sensor coupled to a bone of a patient and the patient condition data includes data indicative of a width of a fracture of the bone.

Preferably, the patient condition sensor includes a physiological sensor and the patient condition data includes physiological data of a patient.

Preferably, the system includes an input peripheral communicatively coupled to the user-worn computer, the user-worn computer being configured to transmit data to the heads-up display in response to commands received from the input peripheral, wherein the input peripheral includes an input peripheral selected from the group consisting of: a microphone, a foot pedal, or a user-worn keyboard.

Preferably, the system includes a remote computer having a peripheral port configured to receive a removable memory device.

Preferably, the remote computer is coupled with at least one of the first processing circuit and the second processing circuit via a network.

Preferably, the first and second processing circuits form a portion of a single computer system.

In another aspect, the invention a method for providing information related to an orthopaedic surgical procedure to a surgeon. The method may include receiving a first signal from a navigation sensor. The method may also include receiving a second signal from a patient condition sensor. The method may further include determining navigational data based on the first signal and determining patient condition data based on the second signal. The method may also include transmitting the navigational data and/or the patient condition data to the user supported computer. The navigational and/or patient condition data may be transmitted using wired or wireless communication. The method may also include displaying the navigational data and/or patient condition data to the surgeon on a heads-up display coupled to the user-worn computer. The method may further include receiving pre-operative data related to the orthopaedic surgical procedure and/or patient from a removable memory device such as a memory device including a flash memory or a microdrive.

Accordingly, the invention also provides a method for providing information related to an orthopaedic surgical procedure to a surgeon, the method comprising:
receiving a first signal from a navigation sensor and a second signal from a patient condition sensor;
determining navigational data based on the first signal and patient condition data based on the second signal;
transmitting the navigational data and the patient condition data to a user-worn computer; and
displaying the navigational data and the patient condition data to the surgeon on a heads-up display coupled with the user-worn computer.

Embodiments of the invention will now be described by way of example with reference to the accompanying drawings, in which:
FIGS. 1 to 5 are simplified block diagrams of different embodiments of a system for providing information related to an orthopaedic surgical procedure to a surgeon; and
FIG. 6 is a simplified flowchart of an algorithm for providing information related to an orthopaedic surgical procedure to a surgeon which may be used by any system of FIGS. 1 to 5.

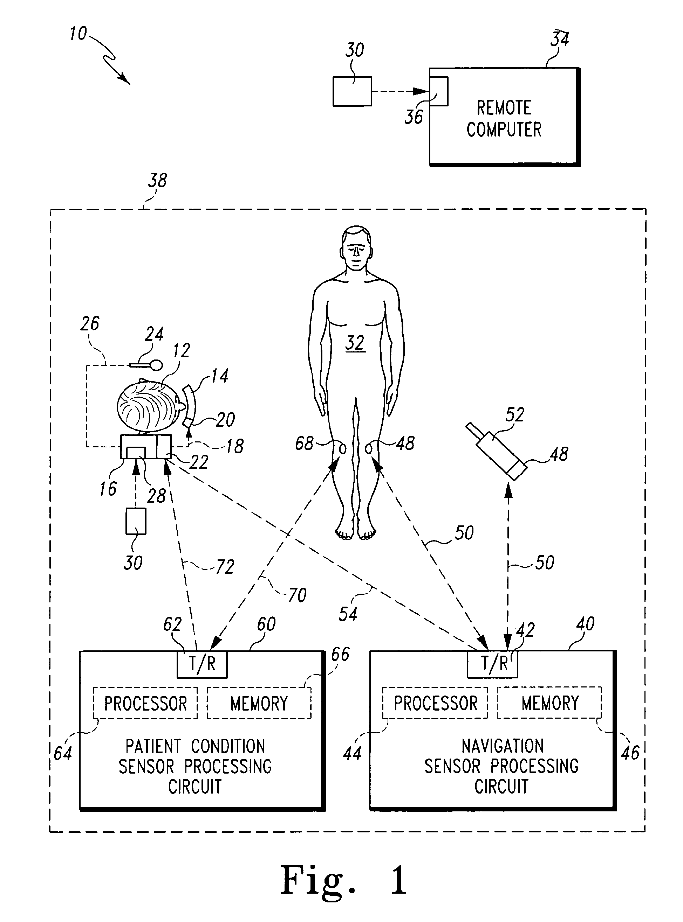

Referring to the drawings, FIG. 1 shows a system 10 for providing information related to an orthopaedic surgical procedure, such as a total knee arthroplasty procedure, to a surgeon 12, which includes a heads-up display 14 and a user-worn computer 16. The heads-up display 14 may be any type of heads-up display configured to be worn on the head or near the head of the surgeon 12. As such, the heads-up display may cover the full field of vision of the surgeon or a portion thereof. The user-worn computer 16 may be any type of computer configured to be worn by the surgeon 12. For example, the user-worn computer 16 may include belts, straps, buttons, and/or other means to support the computer 16 about the waist or on the back of the surgeon 12. Illustratively, the user-worn computer 16 includes devices found in typical computer systems such as a central processing unit, memory, and a display driver configured to operate or communicate with the heads-up display 14 to display images to the surgeon 12.

The heads-up display 14 and the user-worn computer 16 are communicatively coupled via a communication link 18. To do so, the heads-up display 14 includes a receiver 20 and the user-worn computer 16 includes a transmitter or transceiver 22. The communication link 18 may be a wired or a wireless communication link. The user-worn computer 16 may communicate with the head-up display 14 using any suitable wired or wireless communication protocol including, but not limited to, USB, Wireless USB, TCP/IP, Wi-Fi, Bluetooth, Zigbee, and the like. In one particular embodiment, the heads-up display 14 and the user-worn computer 16 are embodied in a wearable computer such as that which is sold by Xybemaut Corporation of Fairfax, Virginia under the trade mark Mobile Assistant V.

The heads-up display 14 and the user-worn computer 16 cooperate to display information related to the surgical procedure to the surgeon 12. In some embodiments, the surgeon 12 may interact with the computer 16 to, for example, request additional images, respond to queries, or the Like, using one of a number of input peripherals such as a handheld, wrist, or user-worn keyboard, a foot pedal, or a microphone. For example, in some embodiments, the system 10 may include a microphone 24 communicatively coupled with the user-worn computer 16 via a communication link 26. The microphone 24 may be any type of microphone or other receiving device capable of receiving voice commands from the surgeon 12. The microphone 24 may be wired (i.e., the communication link 26 may be a wired communication link) or wireless (i.e., the communication link 26 is a wireless communication link). The microphone 24 may be attached to a support structure, such as a ceiling or wall of the operating room, so as to be positionable over the surgical area. Alternatively, the microphone 24 may be appropriately sized and configured to be worn, such as on the surgeons head or clothing, or held by the surgeon 12 or other surgical staff member. For example, in some embodiments, the microphone 24 is an ear or throat microphone. Further, the microphone 24 may be incorporated into the heads-up display 14 or the user-worn computer 16. As such, the term microphone, as used herein, is intended to include any transducer device capable of transducing an audible sound into an electrical signal.

In some embodiments, the user-worn computer 16 may also include a peripheral port 28 configured to receive a removable memory device 30. In the illustrative embodiment, the peripheral port 28 is a Universal Serial Bus (USB) port. However, in other embodiments, the peripheral port 28 may be embodied as any type of serial port, parallel port, or other data port capable of communicating with and receiving data from the removable memory device 30. The removable memory device 30 may be embodied as any portable memory device configured for the purpose of transporting data from one computer system to another computer system. In some embodiments, the removable memory device 30 is embodied as a removable solid-state memory device such as a removable flash memory device. For example, the removable memory device 30 may be embodied as a "memory stick" flash memory device, a flash memory device (as sold under the trade mark SmartMedia), or a flash memory device (as sold under the trade mark CompactFlash). Alternatively, in other embodiments, the removable memory device 30 may be embodied as a memory device having a microdrive for data storage. Regardless, the removable memory device 30 is capable of storing data such as patient condition data for later retrieval.

In use, the surgeon 12 may operate the user-worn computer 16 (e.g., via the microphone 24) to retrieve the data stored on the removable memory device 30. In this way, the surgeon 12 might "call up" or otherwise view pre-operative data that has been previously stored on the removable memory device 30. As used herein, the term "pre-operative data" refers to any data related to the orthopaedic surgical procedure to be performed, any data related to a patient 32 on which the orthopaedic surgical procedure will be performed, or any other data useful to the surgeon 12 during the performance of the orthopaedic surgical procedure. For example, the pre-operative data may include, but is not limited to, historic patient data such as X-rays and medical records, data prepared by the surgeon 12 such as pre-operative notes, diagrams, and surgical plans, and images such as three dimensional rendered images of the relevant anatomical portions of the patient 32 and surgical procedure images illustrating individual steps of the orthopaedic surgical procedure.

In some embodiments, the surgeon 12 may use a remote computer 34 to store the pre-operative data on the removable memory device 30. As such, the remote computer 34 includes a peripheral port 36 configured to receive the removable memory device 30. As used herein the term "remote computer" is intended to refer to any computer or computer system which is not directly communicatively coupled to a network of the healthcare facility. That is, pre-operative data contained in a remote computer is not directly accessible via a network of the healthcare facility. For example, the remote computer 34 may be located in the offices of the surgeon 12, which may not be located at the healthcare facility or hospital at which the orthopaedic surgical procedure is to be performed. As such, the remote computer 34 may not be communicatively linked with computers or data networks of the healthcare facility.

As previously discussed, prior to the performance of the orthopaedic surgical procedure, the surgeon 12 may develop or collect pre-operative data such as pre-operative notes, diagrams, or surgical plans, X-rays, and the medical history of the patient 32. Because the remote computer 34 is not directly linked with a network of the healthcare facility, any pre-operative data stored on the remote computer 34 may not be accessible from the operating room 38. However, the surgeon 12 may store the pre-operative data on the removable memory device 30 using the remote computer 34. Subsequently, during or just prior to the performance of the orthopaedic surgical procedure, the surgeon 12 may couple the removable memory device 30 to the user-worn computer 16 via port 28 and operate the user-worn computer 16 to retrieve the pre-operative data stored on the removable memory device 30. In this way, the surgeon 12 has access to pre-operative data not typically directly accessible in the operating room 38.

The system 10 also includes a navigation sensor processing circuit 40. Illustratively, the processing circuit 40 is located in the operating room 38. The processing circuit 40 includes a transmitter, receiver, or transceiver 42. Additionally, in some embodiments, the processing circuit 40 may include a processor 44 and a memory device 46. In such embodiments, the memory device 46 includes programming code that is executable by the processor 44 to cause the processing circuit 40 to operate in the manner as described hereafter. For example, the processing circuit 40 used in the system commercially available from DePuy Orthopaedics Inc of Warsaw, Indiana under the trade mark Ci.

The navigation sensor processing circuit 40 is configured to receive a data signal from one or more navigation sensors 48 via the transceiver 42. The processing circuit 40 receives the data signal from the navigation sensors 48 via a communication link 50. The communication link 50 may be a wired or a wireless communication link and may use any communication protocol suitable to transmit the data signal from the navigation sensors 48 to the processing circuit 40. As used herein, the term "navigation sensor" refers to any sensor configured to produce a data signal indicative of the location of the sensor or a structure to which the sensor is coupled. For example, in some embodiments, one or more navigation sensors 48 may be implanted into or otherwise coupled with a bone of the patient 32. In such embodiments, the navigation sensors 48 produce a data signal indicative of the relative position of the bone. In other embodiments, a navigation sensor 48 may be coupled with an orthopaedic surgical tool 50 such as a ligament balancer tool. In such embodiments, the navigation sensor 48 produces a data signal indicative of the relative position the surgical tool 50. In yet other embodiments, a navigation sensor 48 may be coupled with or otherwise included in a medical implant such as an orthopaedic implant device. In such embodiments, the navigation sensor 48 produces a data signal indicative of the location of the medical implant.

The navigation sensors 48 may be internally powered (e.g., include a power source such as a battery) or may be externally powered (e.g., receive power from an external source such as an interrogation signal or an electromagnet). As such, in some embodiments, the processing circuit 40 may be configured to generate an interrogation signal to cause one or more of the navigation sensors 48 to produce a data signal, which is subsequently received by the processing circuit 40 via the transceiver 42. Regardless, the processing circuit 40 is also configured to process the data signal received from the navigation sensors 48 to determine navigational data. As used herein, the term "navigational data" refers to any data related to the location of the sensor or structure to which the sensor is coupled and/or to any data derived therefrom such as motion data related to the direction or speed of movement of the sensor 48 or structure. Once the processing circuit 40 has determined the navigational data, the processing circuit 40 is configured to transmit the navigational data to the user-worn computer 16 via the transceiver 42. The user-worn computer 16 receives the navigational data via the transceiver 22 and is configured to automatically or upon request display the navigational data to the surgeon 12 via the heads-up display 14. Accordingly, the navigation sensor processing circuit 40 and the user-worn computer 16 are coupled in communication via a communication link 54. The communication link 54 may be a wired or a wireless communication link and may use any communication protocol suitable to transmit the navigational data from the processing circuit 40 to the user-worn computer 16.

The system 10 further includes a patient condition sensor processing circuit 60. Illustratively, the processing circuit 60 is located in the operating room 38 along with the processing circuit 40. The processing circuit 60 includes a transmitter, receiver, or transceiver 62. Additionally, in some embodiments, the processing circuit 60 may include a processor 64 and a memory device 66. In such embodiments, the memory device 66 includes programming code that is executable by the processor 64 to cause the processing circuit 60 to operate in the manner described hereafter.

The patient condition sensor processing circuit 60 is configured to receive a data signal from one or more patient condition sensors 68 via the transceiver 62. The processing circuit 60 receives the data signal from the patient condition sensors 68 via a communication link 70. The communication link 70 may be a wired or a wireless communication link and may use any communication protocol suitable to transmit the data signal from the patient condition sensors 68 to the processing circuit 60. As used herein, the term "patient condition sensor" refers to any sensor, other than a navigation sensor, configured to produce a data signal indicative of a condition of the patient. For example, in one embodiment, the patient condition sensor 68 may be embodied as a pressure sensor positioned and configured to produce a data signal indicative of a joint pressure between two bones (e.g., the tibia and the femur) of the patient 32. Alternatively, the patient condition sensor 68 may be embodied as a fracture monitoring sensor coupled to a bone of the patient 32 and configured to produce a data signal indicative of a width of the bone facture. As the bone heals, the width of the bone fracture decreases thereby providing data indicative of the healing process of the bone. In other embodiments, the patient condition sensor 68 may be embodied as a physiological sensor positioned and configured to produce a data signal indicative of some type of physiological data related to the patient such as, for example, a heart rate, a blood pressure value, etc.

The patient condition sensors 68 may be internally powered (e.g., include a power source such as a battery) or may be externally powered (e.g., receive power from an external source such as an interrogation signal or an electromagnet). As such, in some embodiments, the processing circuit 60 may be configured to generate an interrogation signal to cause one or more of the navigation sensors 68 to produce a data signal, which is subsequently received by the processing circuit 60 via the transceiver 62. Regardless, the processing circuit 60 is also configured to process the data signal received from the patient condition sensors 48 to determine patient condition data based on the received data signal. As used herein, the term "patient condition data" refers to any data relating to a condition of the patient (i.e., data related to a patient 32 on which the orthopaedic surgical procedure will be performed) including, but not limited, to physiological conditions (e.g., heart rate, blood pressure, etc.) and anatomical conditions (e.g., joint pressure values, bone fracture width values, etc.). Once the processing circuit 60 has determined the patient condition data, the processing circuit 60 is configured to transmit the patient condition data to the user-worn computer 16 via the transceiver 62. The user-worn computer 16 receives the patient condition data via the transceiver 22 and is configured to automatically or upon request display the patient condition data to the surgeon 12 via the heads-up display 14. Accordingly, the patient condition sensor processing circuit 60 and the user-worn computer 16 are coupled in communication via a communication link 72. The communication link 72 may be a wired or wireless communication link and may use any communication protocol suitable to transmit the patient condition data from the processing circuit 60 to the user-worn computer 16.

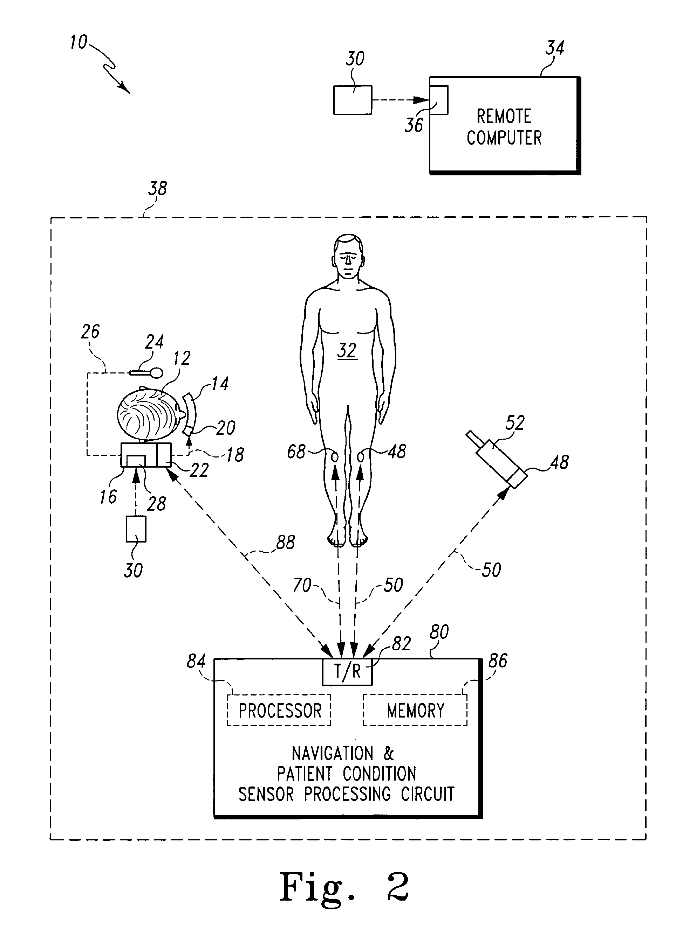

Referring now to FIG. 2, in another embodiment, the navigation sensor processing circuit 40 and the patient condition sensor processing circuit 60 are embodied as a single computer system 80. In such embodiments, the system 80 includes a transmitter, receiver, or transceiver 82 capable of receiving data signals from the navigation sensors 48 and the patient condition sensors 68. Additionally, the computer system 80 may include one or more processors 84 and memory devices 86 as required to process the data signals. As such, the memory device 46 may include programming code that is executable by the processor 44 to cause the processing circuit 40 to operate in the manner as described hereafter.

The computer system 80 is configured to receive the data signals from the navigation sensors 48 and the patient condition sensors 68. The system 80 is also configured to determine navigational data based on the data signals received from the sensors 48 and to determine patient condition data based the data signals received from the sensors 68. The computer system 80 transmits the navigational data and/or the patient condition data to the user-worn computer 16 via the transceiver 82 and over a communication link 88. The communication link 88 may be a wired or a wireless communication link and may use any communication protocol suitable to transmit the navigational data and/or the patient condition data from the computer system 80 to the user-worn computer 16. The user-worn computer 16 receives the transmitted data via the transceiver 22 and is configured to automatically or upon request display the navigational data and/or patient condition data to the surgeon 12 via the heads-up display 14.

In some embodiments, the computer system 80 is configured as a server and the user-worn computer 16 is configured as a client. As such, the computer system 80 may have stored in the memory device 86 and may execute application software such as database programs, word processing programs, or the like. Functions of the application software are accessible by the user-worn computer 16. For example, the surgeon 16 may search and retrieve data from a database stored on the computer system 80 using the user-worn computer 16 as a client. To do so, the surgeon provides a command to the user-worn computer 16 (e.g., via microphone 24). In response to the command, the user-worn computer 16 transmits a database request via transceiver 22 to the computer system 80 over the communication link 88. In response to the database request, the computer system 80 accesses the database and retrieves the requested data. The computer system 80 then transmits the retrieved data to the user-worn computer 16 via the transceiver 82 and over the communication link 88. The user-worn computer 16 may then display the requested data to the surgeon 12 via the heads-up display 14.

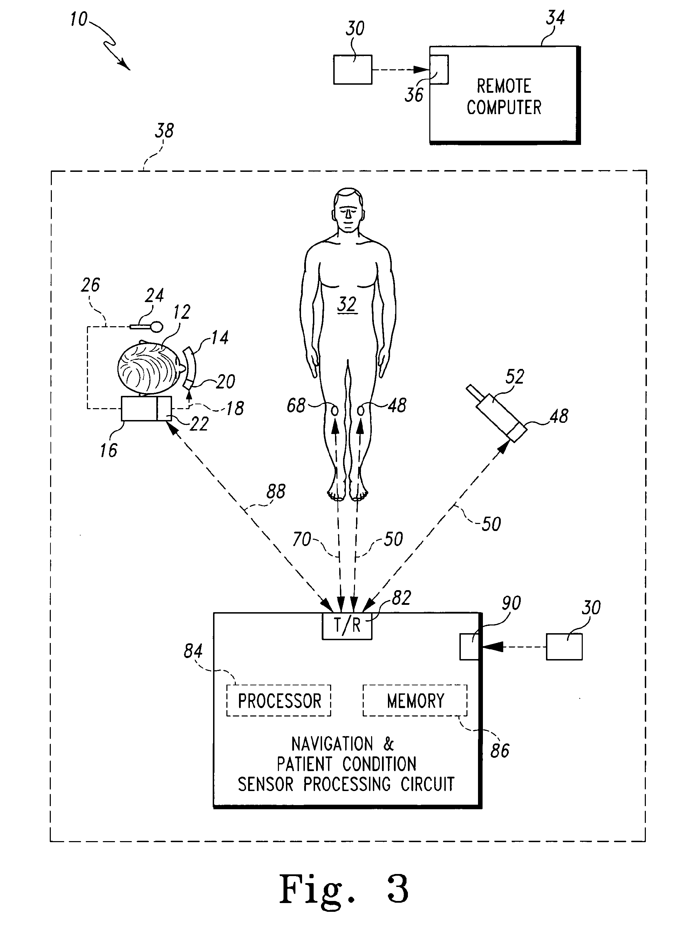

Additionally, as illustrated in FIG. 3, the computer system 80 may include a peripheral port 90 configured to receive the removable memory device 30. In such embodiments, the user-worn computer 16 may or may not include the peripheral port 28. The surgeon 12 may access the patient condition data stored on the removable memory device 30 via the user-worn computer 16. To do so, the surgeon 12 provides a command to the user-worn computer 16 (e.g., via microphone 24). In response to the command, the user-worn computer 16 transmits a request command via transceiver 22 and the communication link 88 to the computer system 80. The request command is received by the transceiver 82 of the computer system 80. In response to the request command, the computer system 80 accesses the removable memory device 30 to retrieve the requested patient condition data. Once retrieved, the computer system 80 transmits the retrieved patient condition data to the user-worn computer 16 via the transceiver 82 and the communication link 88. The user-worn computer 16 may then display the requested patient condition data to the surgeon 12 via the heads-up display 14.

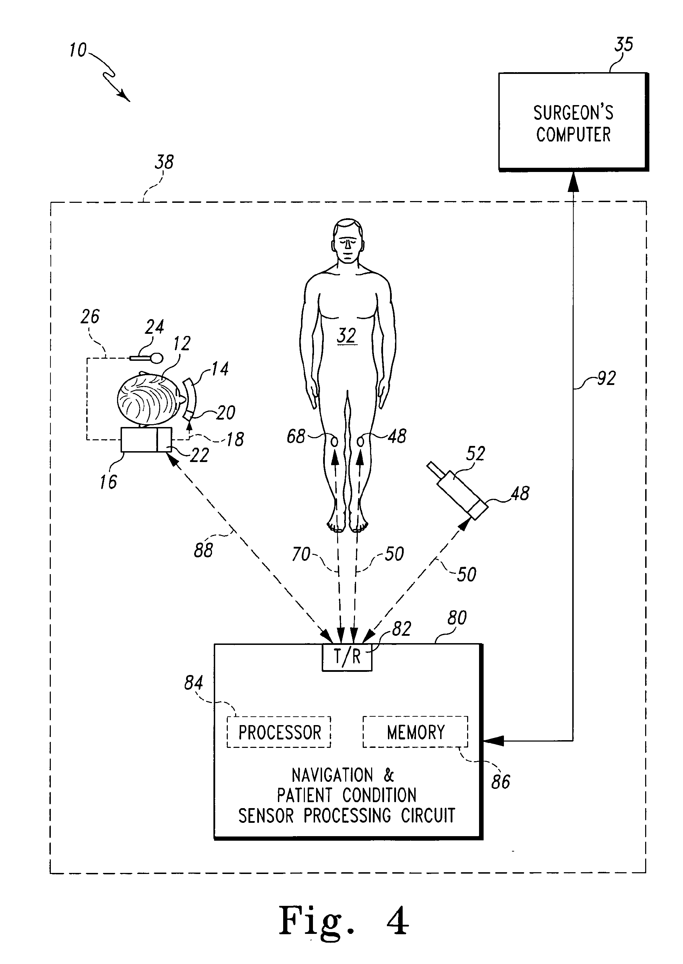

Referring now to FIG. 4, in another embodiment, the pre-operative data developed and/or collected by the surgeon 12 may be stored on a surgeon's computer 35 and accessed via the computer system 80. In such embodiments, the surgeon's computer 35 is communicatively coupled with the computer system 80 via a network link 92. The network link 92 may form portion of a local area network (LAN), a wide area network (WAN), or a publicly-accessible global network. For example, the network link 92 may be embodied as a direct connection between the surgeon's computer 35 and the computer system 80, may form a portion of the healthcare facility's data network, or may form a portion of the Internet. As such, the surgeon's computer 35 and the computer system 80 may include one or more network communication devices, such as Ethernet communication cards, to facilitate communication between the computer 35 and system 80 over the network link 92.

In the embodiment illustrated in FIG. 4, the surgeon 12 may access the pre-operative data stored on the surgeon's computer 35 via the user-worn computer 16 by providing a command to the user-worn computer 16 (e.g., via microphone 24). In response to the command, the user-worn computer 16 transmits a first request command via transceiver 22 and the communication link 88 to the computer system 80. The first request command is received by the transceiver 82 of the computer system 80. In response to the first request command, the computer system 80 transmits a second request command to the remote computer 34 via the network link 92. Once the surgeon's computer 35 receives the second request command, the computer 35 retrieves the requested pre-operative data and transmits the retrieved pre-operative data back to the computer system 80 via the network link 92. The computer system 80 subsequently transmits the retrieved patient condition data to the user-worn computer 16 via the transceiver 82 and the communication link 88. The user-worn computer 16 may then display the requested pre-operative data to the surgeon 12 via the heads-up display 14.

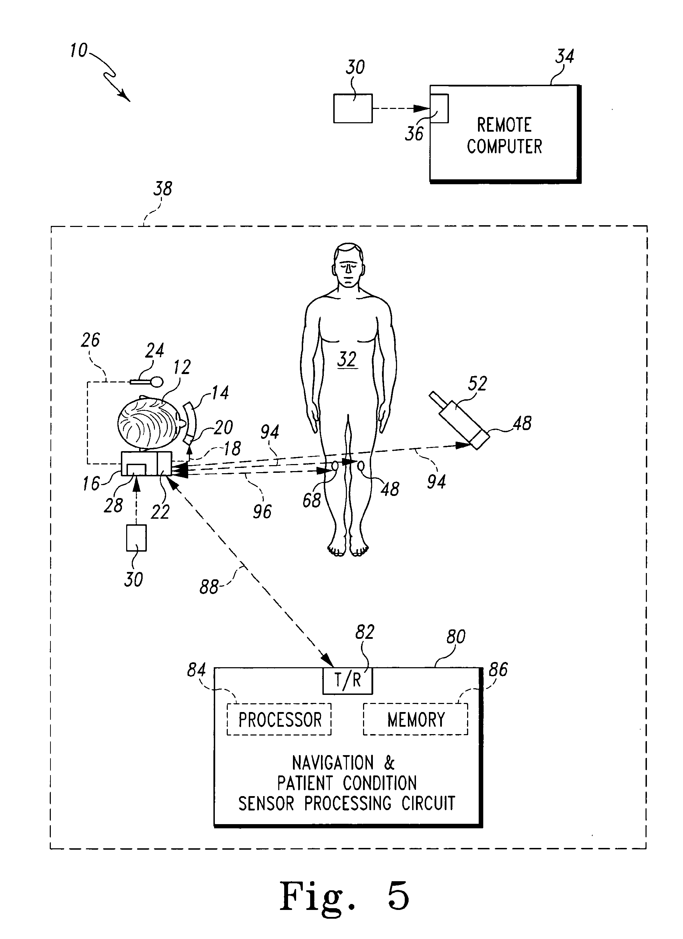

Referring now to FIG. 5, in some embodiments, the user-worn computer 16 is configured to receive the data signals from the navigational sensors 48 and/or the patient condition sensors 68. In such embodiments, the user-worn computer 16 receives the data signals from the navigational sensors 48 via a communication link 94. The user-worn computer 16 receives the data signals from the patient condition sensors 68 via a communication link 96. The communication links 94, 96 may be wired or wireless communication links and may use any communication protocol suitable to transmit the data signals to the user-worn computer 16. In some embodiments, the communication links 94 and 96 form the same communication link. Regardless, the user-worn computer 16 is configured to receive the data signals and transmit the data signals to the computer system 80 via the communication link 88. The computer system 80 processes the data signals to determine navigational data based on data signals received from the navigation sensors 48 and/or patient condition data based on the data signals received form the patient condition sensors 68. The computer system 80 subsequently transmits the navigational data and/or the patient condition data to the user-worn computer 16 via the communication link 88. The user-worn computer 16 may then display the navigational data and/or the patient condition data to the surgeon 12 via the heads-up display 14.

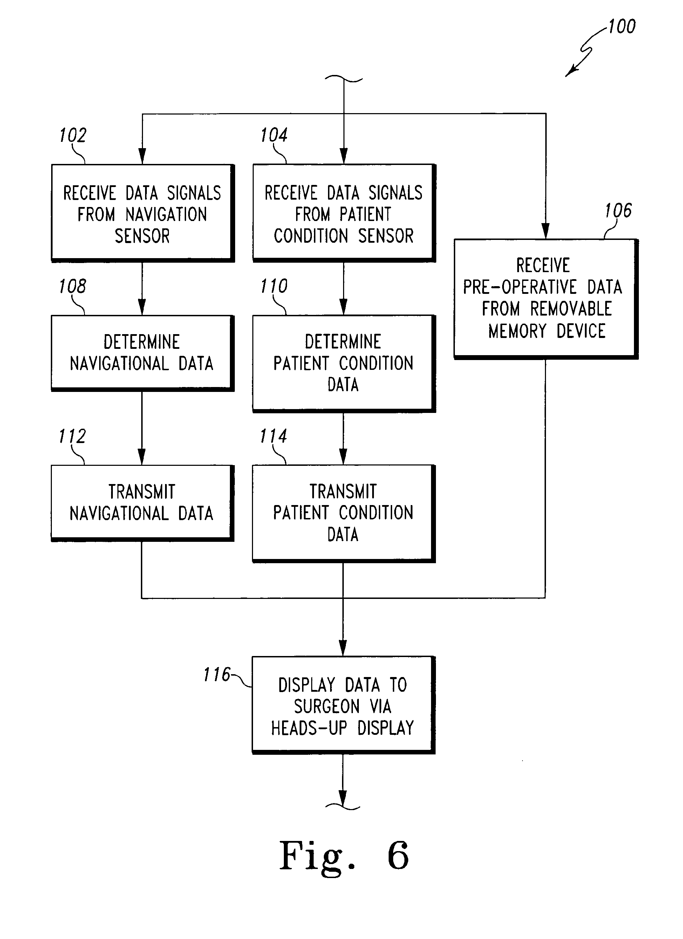

Referring now to FIG. 6, an algorithm 100 for providing information related to an orthopaedic surgical procedure to a surgeon executable by the system 10 is shown. The algorithm 100 includes a process step 102 in which data signals are received from the navigation sensors 48. Depending on the particular embodiment of the system 10, the data signals may be received by the navigation sensor processing circuit 40, the computer system 80, or the user-worn computer 16 (in those embodiments wherein the circuit 40 forms a portion of the computer 16). In process step 104, data signals are received from the patient condition sensors 68. Again, depending on the particular embodiment of the system 10, the data signals may be received by the patient condition sensor processing circuit 60, the computer system 80, or the user-worn computer 16 (in those embodiments wherein the circuit 60 forms a portion of the computer 16). Additionally, in process step 106, pre-operative data is retrieved from the removable memory device 30. Depending on where the removable memory device 30 is coupled, the user-worn computer 16 may retrieve the pre-operative data from the removable memory device 30 as illustrated in and discussed in regard to FIG. 1. Alternatively, the computer system 80 may the retrieve the patient condition data from the removable memory device 30 as illustrated in and discussed in regard to FIG. 3. The process steps 102, 104, and 106 may be executed contemporaneously or sequentially.

In process step 108, navigational data is determined based on the data signals received from the navigation sensors 48. This process step may be performed by the processing circuit 40 or the computer system 80. In process step 110, patient condition data is determined based on the data signals received from the patient condition sensors 68. This process step may be performed by the processing circuit 60 or the computer system 80.

In process step 112, the navigational data is transmitted to the user-worn computer 16. In embodiments wherein the navigational data is determined by the processing circuit 40, the navigational data is transmitted to the user-worn computer 16 via the communication link 54 as illustrated in FIG. 1. In embodiments wherein the navigational data is determined by the computer system 80, the navigational data is transmitted to the user-worn computer 16 via the communication link 88 as illustrated in FIG. 3.

In process step 114, the patient condition data is transmitted to the user-worn computer 16. In embodiments wherein the patient condition data is determined by the processing circuit 60, the patient condition data is transmitted to the user-worn computer 16 via the communication link 72 as illustrated in FIG. 1. In embodiments wherein the patient condition data is determined by the computer system 80, the patient condition data is transmitted to the user-worn computer 16 via the communication link 88 as illustrated in FIG. 3.

Any navigational data and/or patient condition data transmitted in process steps 112, 114, respectively, and/or the pre-operative data received in process step 106 are displayed to the surgeon 12 in process step 116. The navigational data, patient condition data, and pre-operative data are displayed to the surgeon 12 via the heads-up display 14. The surgeon 12 may view the displayed data and interact with the user-worn computer 16 to request additional navigational and/or patient condition data. Depending on the data requested, the algorithm 100 may loop back to process step 102, 104 and/or process step 106 to retrieve the additional navigational, patient condition data, and/or pre-operative data.

## Claims

1. A system for providing information related to an orthopaedic surgical procedure to a surgeon, the system comprising:
a heads-up display configured to be worn by the surgeon;
a user-worn computer configured to be worn by the surgeon and communicatively coupled to the heads-up display, the user-worn computer being configured to receive data via a receiver and display the data to the surgeon on the heads-up display;
a first processing circuit configured to receive a first data signal from a navigation sensor, determine navigational data based on the first data signal, and transmit the navigational data to the user-worn computer, and
a second processing circuit configured to receive a second data signal from a patient condition sensor, determine patient condition data based on the second data signal, and transmit the patient condition data related to the orthopaedic surgical procedure to the user-worn computer.

2. The system of claim 1, wherein the user-worn computer includes a peripheral port configured to receive a removable memory device.

3. The system of claim 1, wherein the navigation sensor is coupled to a bone of a patient and the navigational data is indicative of the location of the bone of the patient.

4. The system of claim 1, wherein the navigation sensor forms a portion of an orthopaedic surgical tool and the navigational data is indicative of a location of the orthopaedic surgical tool.

5. The system of claim 1, wherein the patient condition sensor includes a pressure sensor and the patient condition data includes data indicative of a pressure applied to the pressure sensor.

6. The system of claim 1, wherein the patient condition sensor includes a fracture monitoring sensor coupled to a bone of a patient and the patient condition data includes data indicative of a width of a fracture of the bone.

7. The system of claim 1, wherein the patient condition sensor includes a physiological sensor and the patient condition data includes physiological data of a patient.

8. The system of claim 1, further comprising an input peripheral communicatively coupled to the user-worn computer, the user-worn computer being configured to transmit data to the heads-up display in response to commands received from the input peripheral, wherein the input peripheral includes an input peripheral selected from the group consisting of: a microphone, a foot pedal, or a user-worn keyboard.

9. The system of claim 1, further comprising a remote computer having a peripheral port configured to receive a removable memory device.

10. The system of claim 9, wherein the remote computer is coupled with at least one of the first processing circuit and the second processing circuit via a network.

11. The system of claim 1, wherein the first and second processing circuits form a portion of a single computer system.
